# EUROPEAN PATENT SPECIFICATION

(11) **EP 2 125 051 B1**
(45) Date of publication and mention of the grant of the patent: **02.02.2011**
(21) Application number: 08730792.2
(22) Date of filing: 27.02.2008
(51) Int. Cl.: A61L 27/34

(54) **COATED LENSES**
BESCHICHTETE LINSEN
LENTILLES REVÊTUES

(30) Priority: 28.02.2007 US 892024 P
(43) Date of publication of application: 02.12.2009
(73) Proprietor: Alcon, Inc., 6331 Hünenberg (CH)
(72) Inventor: JINKERSON, David, L., Benbrook, Texas 76126 (US); KARAKELLE, Mutlu, Fort Worth, Texas 76132 (US)
(74) Representative: Teipel, Stephan
(86) International application number: PCT/US2008/055059
(87) International publication number: WO 2008/106477

(56) References cited:
- EP-A- 0 809 997
- EP-A- 0 963 761
- EP-A- 1 535 952
- WO-A-01/09646
- US-A- 5 080 924

## Description

### TECHNICAL FIELD

The embodiments described herein generally relate to intraocular lenses and other medical implants coated with a composition that minimizes the adherence of cellular growth and/or proteins to coated surfaces.

### BACKGROUND

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens. When trauma, age or disease cause the lens to become less transparent, vision deteriorates because of the diminished amount of that can be transmitted to the retina. This deficiency in the lens of the eye is medically known as a cataract. The treatment for this condition is surgical removal of the lens and implantation of an artificial lens known as an intraocular lens or "IOL."

In general, the procedures for cataracted lens removal and IOL implantation have become common place and virtually routine. However, in some instances, after IOL implantation, cellular proliferation takes place on the rear of the capsular membrane. This condition is known as secondary cataract formation or more accurately as posterior capsular opacification because the cellular growth tends to block light transmission to the retina causing vision to deteriorate. Typical treatment involves the periodic use of Nd:YAG laser light to ablate the cellular growth from posterior lens capsule surface. During the ablation process, a portion of the capsular membrane at the rear of the lens is also affected. The membrane may be punctured and this may result, at a minimum, in the exposure of the rear of the lens to the vitreous of the eye. The vitreous may infiltrate past the lens into the aqueous, which is undesirable. Accordingly, the procedure poses issues. In addition, the periodic nature of this treatment imposes inconvenience on the patient by requiring frequent office visits.

Posterior capsular opacification appears to be dependent on a number of factors, some patient-related and some IOL-related. Some IOLs appear to be less prone to posterior opacification than others. Pharmacological approaches to prevent or inhibit posterior capsular opacification have been explored and some approaches have included cytotoxic agents in solution or for release from surfaces of an IOL into surrounding fluid and tissue. However, such a free cytotoxic agent may have deleterious effects on other intraocular tissue.

Cellular proliferation and protein adhesion are not limited to implanted IOLs but occur fairly frequently when other devices are implanted into a patient. For example, medical devices such as shunts (used in dialysis treatment, or for long term routine intravenous administration of medications and/or nutrients, for example), glaucoma shunts, pace makers, defibrillators, cardiac stents, and the like, also often experience cellular proliferation and protein adhesion on surfaces. Such cellular growth and protein adhesion can pose significant issues. For example, a dialysis shunt might have to be cleaned periodically to remove adhering protein and/or cellular growth and might ultimately have to be removed and replaced. When it becomes necessary to replace such a shunt due to tissue blockage, the new shunt must usually be installed in a different blood vessel at a different site. A patient has a limited number of suitable sites for shunts. Accordingly, the blocking of shunts with cellular and/or protein tissue poses a serious issue in prolonged patient care.

One of the primary areas of concern in the use of re-usable contact lenses (i.e. not the single-use disposable lenses) is maintaining a clean lens surface. In ordinary use, the contact lenses will gradually become encrusted with protein matter that at a minimum affects wearer comfort and that may in some cases lead to more serious issues. Accordingly, users are advised to clean lenses at intervals, such as daily, according to a protocol that is designed to remove these protein deposits. Failure on the part of a significant proportion of users to follow the cleaning protocols precisely or to regularly carry these out as recommended may in some cases lead to complications.

Accordingly, it is desirable to develop a coating for medical implants such as IOLs, contact lenses, shunts, pace makers, defibrillators, and the like that inhibits or prevents the adhesion of proteins and cellular proliferation on the coating. In addition, it is desirable in the case of IOLs and contact lenses that the coating has good optical light transmission properties. Furthermore, other desirable features and characteristics of the coated IOLs, contact lenses and other medical implants will become apparent from the subsequent detailed description and the appended claims, taken in conjunction with the accompanying drawings and the foregoing technical field and background.

### BRIEF SUMMARY

The invention provides a coated medical implant according to claim 1. The medical implant has an implant surface and a coating is formed on at least a portion of the implant surface. The coating includes a coating outer surface of a first chemical component that is chemically bonded to a carboxylate functionality of a second chemical component. The second chemical component is immobilized by amide linkage to an underlying third chemical component that is plasma coated directly onto implant body surfaces. The coating inhibits or prevents the adhesion of protein and/or cellular proliferation on the coated portion of the implant surface.

The optically transparent lens body has an optically clear coating formed on at least a portion of the lens body surface that inhibits protein adhesion and cellular adhesion to the lens body. The coating includes a coating outer surface of a first chemical component that is chemically bonded to a carboxylate functionality of an organic acid. The organic acid has an average molecular weight in a range from about 2,000 to about 10,000 and is immobilized by amide linkage to an underlying second chemical component. The second chemical component is plasma coated directly onto the lens body surface.

### BRIEF DESCRIPTION OF THE DRAWINGS

Various embodiments will hereinafter be described in conjunction with the following schematic, not-to-scale drawing figures, wherein like numerals denote like elements, and
FIG. 1 is an example of an embodiment of a coated medical implant of the invention;
FIG. 2 is a cross sectional view of a portion of the medical implant of FIG. 1 schematically depicting an example of an embodiment of a coating; and
FIG. 3 is a flow diagram of an exemplary embodiment of a method of the invention for making coated medical implants.

### DETAILED DESCRIPTION

The following detailed description is merely exemplary in nature and is not intended to limit the described embodiments or the application and uses of the described embodiments. Furthermore, there is no intention to be bound by any expressed or implied theory presented in the preceding technical field, background, brief summary or the following detailed description.

In the following description and claims the term "amine" should be broadly read to include all those chemical compounds that include a (-C-NH2) group, a (-C-NHR) group or a (-C-NR2) group where R = an alkyl or aryl group.

In the following description and claims the term "chemical component" means a chemical compound that may be chemically bound to other chemical compounds to form a coating. Thus, each chemical compound may be a "chemical component" of the coating. Numbering of chemical components as "first," "second," or "third" has no significance other than to distinguish one from the other.

In the following description and claims the term "medical implant" includes intraocular lenses ("IOLs") and contact lenses. While the latter may not be permanently implanted, during use they are in direct contact with body tissue (the cornea) and fluids (tears).

In the following description and claims the term "polyacrylic acid" should be broadly read to include polymers that have at least two carboxylate groups.

While the discussion that follows focuses primarily on IOLs for convenience and brevity, it should be understood that the technology applies to other medical implants as well.

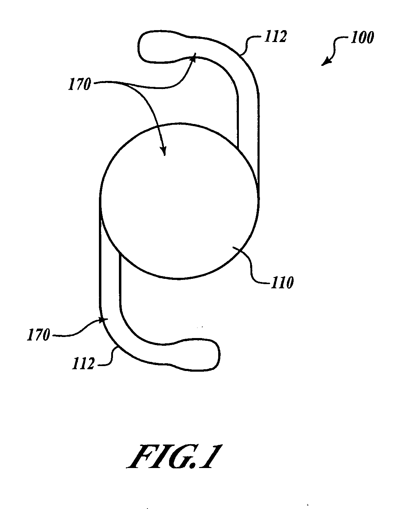

An example of an embodiment of a coated IOL 100 of the invention is shown in FIG. 1. In this example, the IOL 100 has a lens body 110 from which a pair of lens retaining structures 112 extend, shown as haptics 112. A coating 170 covers the haptics 112 and the lens body 110.

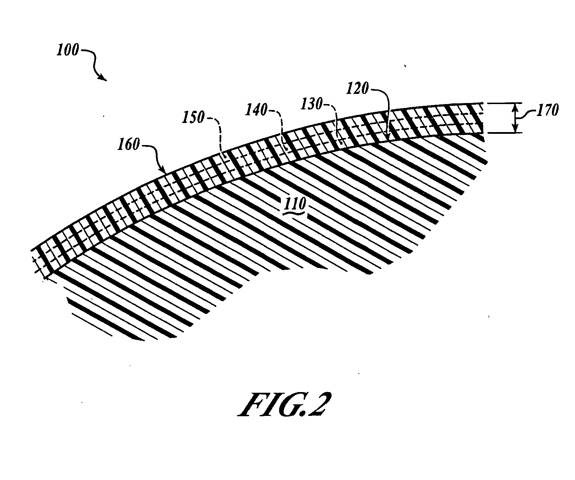

A portion of a cross section through lens body 110 is shown in FIG. 2. This schematic representation of the coating 170 depicts distinct layers for explanatory purposes only. The coating depicted in FIG. 2 will not appear as separate and distinct layers under magnification because, once chemically bonded to each other, separate chemical reactants are not usually visible as separate layers, but only as a single layer. Briefly, coating 170 is illustrated as including three layers or chemical components. A first chemical component 130 is directly plasma coated onto the outer surface 120 of lens body 110. A second chemical component 140 is chemically bonded to the first chemical component by amide linkage. A third chemical component 150 is chemically bonded to the second chemical component 140 by linkage to free carboxylate groups of the second chemical component. The third chemical component 150 presents an outer surface 160 that is exposed to the surrounding environment Appropriate selection of the third chemical component customizes the outer surface properties for a selected intended purpose, for example, repelling proteins or inhibiting cellular adhesion.

The first chemical component 120 is selected from the chemical group of amines. Desirably, the selected amine should be relatively volatile for ease of deposition directly onto the implant surface by RF ("radio frequency") plasma vapor deposition or chemical vapor deposition techniques. These techniques facilitate chemical reaction between the amine and the surface of the implant to provide a tightly adhering thin amine film on the surface of the implant The selected amine should further have at least one free (-C-NH2) group or (-C-NHR) group (where R = an alkyl or aryl group) that is available for reaction after the amine film is deposited. Amine films may be deposited by plasma techniques on materials used to form IOLs and contact lenses, such as soft acrylic materials, silicone-type polymers, polymethylmethacralate and its derivatives, and the like. In addition, amine films may be deposited onto organic polymers used to form other implants such as dialysis shunts, glaucoma shunts, and the like. Further, amine films may be deposited onto metals typically used in defibrillators, pacemakers, cardiac stents, and the like. A non limiting list of examples of useful first chemical components includes: heptylamine, allylamine, 2-amino-methacralate, 2-amino-ethylmethacralate, amino-ethylene, ethylamine, hexylamine and the like. Primary and secondary amines are preferred but others may also be used In general, the plasma-deposited film thickness is of the order of about 10 to about 300 Angstroms, but other thicknesses may also be useful.

Once the first chemical component has been deposited as a thin film on the medical implant outer surface, a second chemical component is reacted with the free amine groups of the thin film. This reaction may be carried out by dipping the plasma coated medical implant into a solution of the second chemical component, or by spin coating, painting or spraying with the solution, or another suitable technique. The second chemical component may be selected from those compositions that are able to chemically bond to free amine groups of the first chemical component, and that have at least one free carboxylate group available for bonding to a third chemical component, after bonding with the first chemical componcnt. Desirably, the second chemical component is selected from organic polymeric acids, such as the polyacrylates that have an average molecular weight in the range from about 2,000 up to about 10,000. This range of average molecular weights is suitable for forming transparent coatings that have appropriate optical properties (e.g. maintains an acceptable degree of optical resolution of images) for use in implants such as IOLs and contact lenses. If the applied coating yet maintains an acceptable image resolution, its effect on image quality may be regarded as "insignificant.". A non limiting list of examples of useful second chemical components includes: carboxylate-containing polysaccharides (e.g. hyaluronic acid, heparin, chondroitin sulfate, carboxymethyl cellulose), polyacrylic acids and esters and derivatives of such acids, , polymaleic acid and acid anhydrides of polymeric carboxylic acids and the like whether natural or synthetic. Non limiting examples of derivatives of acids include polymaleic anhydride, and copolymers of carboxylate containing monomers, such as, acrylic acid, methacrylic acid, maleic acid and maleic anhydride with other non-carboxylic acid monomers, like methyl methacrylate.

The chemical combination of the first and second chemical components and immobilization of the reaction product on the medical implant surface provides a platform for adding a selected third chemical component. The third chemical component should include moieties that are able to chemically react with free carboxylate groups of the second chemical component. Accordingly, the third chemical component may be selected from a wide range of chemical compositions, and is primarily selected based upon the desired nature of the coating surface. For example, the third chemical component may form a cell-disrupting coating. In the case of a cell-disrupting coating, the third chemical component may include, for example, an amino acid or a lytic peptide for an IOL to prevent posterior capsular adhesion. The third chemical component may also bc, for example, in the case of an IOL or contact lens, any of melattin, selenosystamine (in a combination produced by interaction with glutathione that is naturally present in the eye), polyhexamethylene biguanide (PHMB), lytic peptides, and the like for inhibiting protein adhesion and cellular growth. In addition to the foregoing and other cell-disrupting coatings, other potential coatings include biocompatible coatings, for example RGDs such as Arg-Gly-Asp-Ser peptide, other amino acids and peptides, proteins such as fibronectin and albumin; and non-fouling coatings, such as polyethylene oxide (PEO), and the like.

Once the third chemical component is applied to the first two and chemically bonded to the carboxylate group by an amide linkage, the coated medical implant is essentially ready for use, after any necessary or required preimplantation procedures, for example, sterilization.

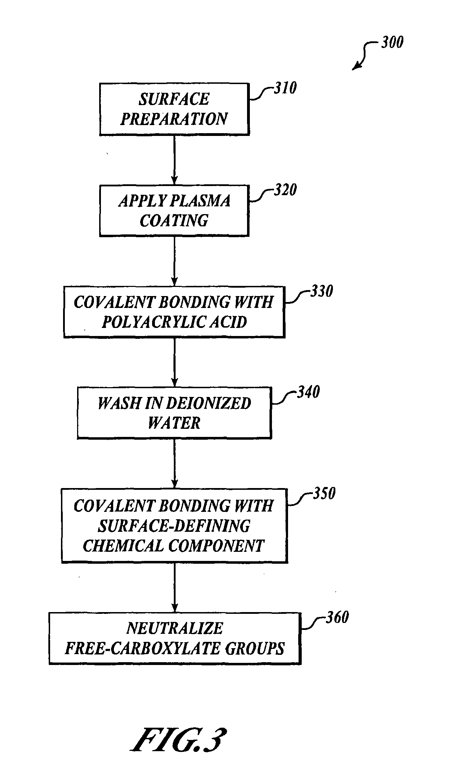

FIG. 3 illustrates an exemplary embodiment of a multi-step process 300 for making coated medical implants. In process 310, the implant surface is prepared for subsequent plasma deposition of amines thereon. The implant surface preparation includes cleaning of dust and any loose debris, degreasing, washing in a suitable detergent and the like. When the surface has been cleaned, it may be dried. The cleaned and dried implant may then be placed in a plasma chamber for plasma coating, in process 320. Plasma coating parameters will depend upon the nature of the first chemical component selected for plasma deposition onto the implant surface.

Plasma coating parameters depend upon the nature of the first chemical component selected for plasma deposition onto the implant surface. Typically, the plasma deposition may be preceded by a plasma cleaning step with argon or oxygen. Suitable amine compounds are gases like ammonia or methylamine, or more commonly, liquid amine compounds, for example, alkylamines, such as, propylamine, butylamine, pentylamine, hexylamine, heptylamine, octylamine, ethylenediamine, and the like. Preferred alkylamines are those of sufficient volatility to readily evaporate in a plasma chamber under vacuum. Of these liquid alkylamine compounds, pentylamine, hexylamine, and heptylamine are preferred, but of these heptylamine is the most preferred. Less volatile amines may also be used by heating the amine compound under vacuum to provide sufficient vapor into the chamber to sustain a plasma.

The amine compounds that have ethylenically unsaturated moieties in their structure, such as olefinic amines, acrylic amines and styrenic amines, are also useful and desirable to utilize. Olefinic amine compounds that are suitable include those which are volatile liquids, such as allylamine, diallylamine or 4-aminobutene. Acrylic amines that are suitable include 2-aminoethylacrylate, 2-aminoethylmethacrylate, 3-aminopropylacrylate, and 3-aminopropylmethacrylate, and the like. An example of a suitable styrenic amine includes 4-aminostyrene.

Plasma deposition can be performed after induction of the organic amine containing compound in vapor form into the chamber. For example, deposition by RF plasma of the organic amine compound can be performed at nominal RF powers, for example in the range from about 30 to about 120 Watts (W) and under chamber pressure which may vary depending on the compound chosen. Typical conditions used for heptylamine may include an RF power of about 60 W at a chamber pressure of about 25 to about 325 mTorr, more typically 110 to 130 mTorr. Coating may be deposited to a coating thickness of 200 Angstroms. Another organic amine compound, allylamine, might be deposited in a RF plasma process at a power of about 100 to 150 W, pressure of about 100-300 mTorr to a thickness of 100 to 500 Angstroms. Accordingly, the conditions of RF plasma deposition may vary based on the particular amine compound selected.

After plasma coating, the second or bridging chemical component may be covalently bonded to free amine groups on the plasma coated surface, in process 330. In general, the bridging chemical may include a polyacrylic acid, and its reaction with free amine groups to form amide linkages may be catalyzed with ethyl dimethyl propyl amino diimde ("EDC"), although other catalysts may also be used. Once the amide linkages are formed, the implant surfaces are washed in deionized water, in process 340. The coated implant surfaces now provide a platform for covalent bonding of a third chemical component thereto by via with free carboxylate groups of the polyacrylic acid.

In process 350 the third chemical component is reacted with at least some of the free reactive carboxylate groups to form a surface coating. The parameters of the carboxylate linkage reaction are dependent upon the particular third chemical component selected, any catalyst used, and other factors ordinarily considered for forming covalent or ionic linkages to carboxylate groups. Once the reaction is complete, then in process 360, any residual free carboxylate groups may be neutralized to produce a useful coated implant, such as an IOL.

The following examples are provided to illustrate at least some embodiments of the invention, and do not limit the scope of the invention as set forth herein and in the appended claims.

### EXAMPLES

### Application of a Heptylamine Film by Plasma Deposition

An RF plasma chamber (Advanced Surface Technology, Inc.) was prepared for materials processing by first performing an oxygen etch to clean the chamber. The oxygen etch was performed by setting the oxygen flow to 50 cc/min with at pressure of 250 mTorr and RF power of 160 W. The oxygen plasma formed had a reflected RF power of no more than 3 W and a characteristic hazy blue color that eventually diminished to a blue-gray color over time. The oxygen etch was continued for 2 hours for chamber cleaning.

Further cleaning of the lens holder plate and thickness gauge was performed in an argon plasma etch. Thereafter, the stainless steel lens holder plate and thickness gauge were loaded into the center of the chamber and thickness gauge electrical leads connected to the chamber control system. Then, the thickness gauge was mounted on the lens holder plate. An argon plasma etch was performed at 140W RF power, 250 mTorr pressure for 30 minutes with argon flow at 90 cc/min. The argon plasma provided a pink to purple color with a reflected RF power of no more than 3 W. After cleaning, the lens holder plate and thickness gauge were removed from the chamber and placed in a laminar flow hood.

After cooling the lens holder plate to ambient temperature, up to 30 ACRYSOF® (Trademark of Alcon, Fort Worth, Texas) intraocular lenses ("IOLs"), Model MA60BM were placed on the lens holder plate. The lens-containing lens holder plate was then loaded into the plasma chamber and the thickness gauge remounted onto the lens holder plate. First, an argon plasma etch was performed on the IOLs in the chamber at RF power of 60 W, 250 mTorr pressure, and argon flow of 90 cc/min. After 6 minutes of argon plasma treatment the RF power was turned off.

Afterwards, a heptylamine plasma coating was applied to the surface of the IOLs in the chamber. Five grams of heptylamine was placed into a 250 mL round-bottomed flask and a fresh single-holed rubber stopper inserted into the flask. The flask interior communicated with the plasma chamber inlet through the holed stopper. The chamber was evacuated for 1 minute and then the needle valve to the heptylamine flask was opened. Evacuation was continued for 3 minutes, then the system was allowed to equilibrate for 10 minutes. The thickness gauge was zeroed and the RF power turned on. The heptylamine plasma deposition was carried out at 60 W until the heptylamine was deposited to a thickness of 200 Angstroms. Under these conditions typical chamber pressures are in the range from about 10 to 50 mTorr. After the desired thickness was achieved the heptylamine flow was stopped and the RF power was turned off. After 2 minutes the chamber was evacuated to remove residual heptylamine. After 10 minutes the chamber was flushed with argon and opened. The IOLs were removed and the lens holder plate placed into a laminar flow hood. The IOLs were labeled according to position by row and column on the lens holder plate. Sessile drop contact angle measurements were performed with water on the heptylamine plasma coated IOL. Typical contact angles were found in the range from 70 to 90o.

### Covalent Bonding of Polyacrylic Acid to Plasma Deposited Film

Each coated IOL was placed in a separate 1.5 ml centrifuge vial which was charged with 0.5 ml 0.012% polyacrylic acid with an average molecular weight of 2,000. To each vial was added 0.1 ml of a fresh 0.4M ethyl dimethyl propyl amino diimde ("EDC") in a pH 3.6 buffered solution. Each closed vial was then mixed on a vortex mixer for about 10 seconds. The vials were allowed to stand for about 1 hour at room temperature to permit further reaction between carboxylate groups of the polyacrylic acid with amine groups to form amide linkages. Four more EDC additions were performed at one hour intervals. After the fifth EDC addition, the lenses each soaked for a further one hour at room temperature. It was observed that upon adding EDC, the solutions in the vials became cloudy and the cloudiness dissipated in about an hour (i.e. prior to the next EDC addition) After standing for about 65 hours at room temperature, the polyacrylic acid was immobilized and the solutions in the vials had a pH of about 3.29. The IOLs were transferred to labeled tissue capsules and the capsules were placed in a 1 liter flask and washed with 600 ml deionized water at 10 minute intervals at room temperature by shaking on a shaker at 100 rpm. After washing, the IOLs were dried in air overnight. The dried IOLs appeared optically clear and transparent. Measurement of contact angle on the coated and dried lenses was performed using an AST Contact Angle VCA 2500 instrument. The results indicate a hydrophobic contact angle of between about 40° to about 60°.

### PHMB Immobilization to Heptylamine/Polyacrylic Acid Surfaces

Into each of several microcentrifuge vials was added 0.25 ml of a 20% PHMB solution as Cosmocil™ QC reagent [Zeneca Biocides, Wilmington, DE] and 0.75 ml of 0.2M sodium phosphate buffer (pH 3.6). Each IOL was removed from its tissue capsule and placed into its respective microcentrifuge vial. To each vial was added 0.1 ml of fresh 0.4 M EDC reagent solution, and the vials were then closed and mixed in a vortex mixer for 10 seconds. The reaction of residual carboxylate groups on the IOL surface was continued for an hour at room temperature to form an amide by reaction with terminal groups on the PHMB molecule. Four more EDC additions were made at one hour intervals for a total of five additions. After the last addition of EDC, the IOLS were allowed to soak at room temperature for about 17-18 hours.

Fresh microcentrifuge vials were prepared, as above, and the IOLs were each transferred to its respective fresh vial. Treatment with EDC was carried out again as before. After the fifth EDC addition and soaking for about 16-17 hours, the vial solutions had a pH of about 4.68.

The IOLs were each transferred back their respective tissue capsules and the tissue capsules were placed in a 600 ml beaker and washed 10 times, while shaking at 100 rpm, in 400 ml deionized water that had been filtered through a 0.2 micron sterile filter. After washing, each IOL was removed from its tissue capsule and placed in a microcentrifuge vial containing 1.0 ml of pH 7.47 Dulbecco's phosphate buffered saline (DPBS) solution which contained about 0.01M phosphate in buffered saline to neutralize any unreacted carboxylate groups. This neutralization continued for 18 hours at room temperature. After neutralization, each IOL was transferred back to its tissue capsule. The pH of the DPBS solution after neutralization was found to be about 7.27. After a further washing in deionized water, the coated IOLs were allowed to dry overnight under ambient conditions.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the described embodiments in any way. Rather, the foregoing detailed description will provide those skilled in the art with a convenient road map for implementing the exemplary embodiment or exemplary embodiments. It should be understood that various changes can be made in the function and arrangement of elements without departing from the scope as set forth in the appended claims and the legal equivalents thereof.

## Claims

1. A coated medical implant comprising:
an optically transparent lens body comprising lens outer surfaces; and
a coating formed on at least a portion of the lens outer surfaces, the coating comprising a coating outer surface comprising a third chemical component, the third chemical component bonded by an amide linkage to a carboxylate functionality of a second chemical component having an average molecular weight in a range from about 2,000 to about 10,000, the second chemical component immobilized by an amide linkage to an underlying first chemical component comprising an amine that is plasma coated directly onto the at least a portion of the lens outer surfaces.

2. The medical implant of Claim 1, wherein the underlying first chemical component comprises at least one of heptylamine, allylamine, diallylamine, 2-amino-methacrylate, 2-amino-ethylmethacralate, amino-ethylene, ethylaminc, ethylenediamine and hexylamine.

3. The medical implant of Claim 1 or 2, wherein the second chemical component comprises at least one of carboxylate-containing polysaccharides, the polyacrylic acids and esters and derivatives of such acids.

4. The medical implant of Claim 1, 2 or 3, wherein the third chemical component is selected from amino acids, lytic peptides, selenosystamine, polyhexamethylene biguanide, proteins and polyethylene oxide.

5. The medical implant of any of Claims 1-4, wherein the medical implant comprises an intraocular lens, and wherein the coating has an insignificant effect on image resolutian of the intraocular lens.

6. The medical implant of any of Claims 1-5 wherein the third chemical component is selected such that the coating comprises one of cell-disrupting coatings, biocompatibilizing coatings, or non-fouling coatings.

7. The medical implant of any of Claims 1-6 wherein the first chemical component comprises a primary or secondary amine.

8. The medical implant of any of Claims 1-7 wherein the coating further comprises residual catalyst comprising ethyl-dimethyl propyl-amino carbo-diimide.

## Patentansprüche

1. Beschichtetes medizinisches Implantat, umfassend:
einen optisch transparenten Linsenkörper, der Linsenaußenflächen umfasst; und
eine Beschichtung, gebildet auf zumindest einem Teil der Linsenaußenflächen, wobei die Beschichtung eine Beschichtungsaußenfläche umfasst, die eine dritte chemische Komponente umfasst, wobei die dritte chemische Komponente durch eine Amidverknüpfung an eine Carboxylat-Funktionalität einer zweiten chemischen Komponente mit einem mittleren Molekulargewicht im Bereich von etwa 2.000 bis etwa 10.000 gebunden ist, wobei die zweite chemische Komponente durch eine Amidverknüpfung an eine darunterliegende erste chemische Komponente, umfassend ein Amin, die direkt auf zumindest einen Teil der Linsenaußenflächen plasmabeschichtet ist, immobilisiert ist.

2. Medizinisches Implantat nach Anspruch 1, wobei die darunterliegende erste chemische Komponente mindestens eines von Heptylamin, Allylamin, Diallylamin, 2-Amino-methacrylat, 2-Amino-ethylmethacrylat, Aminoethylen, Ethylamin, Ethylendiamin und Hexylamin umfasst.

3. Medizinisches Implantat nach Anspruch 1 oder 2, wobei die zweite chemische Komponente mindestens eines von Carboxylat-enthaltenden Polysacchariden, Polyacrylsäuren und Estern und Derivaten dieser Säuren umfasst.

4. Medizinisches Implantat nach Anspruch 1, 2 oder 3, wobei die dritte chemische Komponente ausgewählt ist aus Aminosäuren, lytischen Peptiden, Selenocystamin, Polyhexamethylenbiguanid, Proteinen und Polyethylenoxid.

5. Medizinisches Implantat nach einem der Ansprüche 1 bis 4, wobei das medizinische Implantat eine intraokulare Linse umfasst und wobei die Beschichtung eine unbedeutende Wirkung auf die Bildauflösung der intraokularen Linse hat.

6. Medizinisches Implantat nach einem der Ansprüche 1 bis 5, wobei die dritte chemische Komponente so gewählt ist, dass die Beschichtung eine von zellzerstörenden Beschichtungen, biologisch verträglich machenden Beschichtungen oder nicht faulenden Beschichtungen umfasst.

7. Medizinisches Implantat nach einem der Ansprüche 1 bis 6, wobei die erste chemische Komponente ein primäres oder sekundäres Amin umfasst.

8. Medizinisches Implantat nach einem der Ansprüche 1 bis 7, wobei die Beschichtung ferner restlichen Katalysator umfasst, der Ethyldimethylpropylaminocarbodiimid umfasst.

## Revendications

1. Implant médical revêtu comprenant :
un corps de lentille optiquement transparent comprenant des surfaces externes de lentille ; et
un revêtement formé sur au moins une partie des surfaces externes de lentille, le revêtement comprenant une surface externe de revêtement comprenant un troisième composant chimique, le troisième composant chimique lié par une liaison amide à une fonctionnalité carboxylate d'un deuxième composant chimique ayant un poids moléculaire moyen dans une plage d'environ 2000 à environ about 10 000, le deuxième composant chimique immobilisé par une liaison amide à un premier composant chimique sous-jacent comprenant une amine qui est directement revêtue de plasma sur la au moins une partie des surfaces externes de lentille.

2. Implant médical selon la revendication 1, dans lequel le premier composant chimique sous-jacent comprend au moins un des composés suivantes : heptylamine, allylamine, diallylamine, 2-amino-méthacrylate, 2-aminoéthylméthacrylate, amino-éthylène, éthylamine, éthylènediamine et hexylamine.

3. Implant médical selon la revendication 1 ou 2, dans lequel le deuxième composant chimique comprend au moins l'un des polysaccharides contenant du carboxylate, des acides polyacryliques et des esters et dérivés de ces acides.

4. Implant médical selon la revendication 1, 2 ou 3, dans lequel le troisième composant chimique est sélectionné parmi les acides aminés, les peptides lytiques, la sélénocystamine, le poly(biguanide d'hexaméthylène), les protéines et le poly(oxyde d'éthylène).

5. Implant médical selon l'une quelconque des revendications 1 à 4, dans lequel l'implant médical comprend une lentille intraoculaire et dans lequel le revêtement a un effet insignifiant sur la résolution de l'image de la lentille intraoculaire.

6. Implant médical selon l'une quelconque des revendications 1 à 5, dans lequel le troisième composant chimique est sélectionné de sorte que le revêtement comprenne l'un des revêtements de rupture cellulaire, des revêtements de biocompatibilisation ou des revêtements résistant à l'encrassement.

7. Implant médical selon l'une quelconque des revendications 1 à 6, dans lequel le premier composant chimique comprend une amine primaire ou secondaire.

8. Implant médical selon l'une quelconque des revendications 1 à 7, dans lequel le revêtement comprend un catalyseur résiduel comprenant de l'éthyldiméthylpropylaminocarbodiimide.
